# EUROPEAN PATENT SPECIFICATION

(11) **EP 0 575 776 B1**
(45) Date of publication and mention of the grant of the patent: **31.03.1999**
(21) Application number: 93108659.9
(22) Date of filing: 28.05.1993
(51) Int. Cl.: C07D 295/08

(54) **A process for the preparation of enantiomers of dropropizine**
Verfahren zur Herstellung von Enantiomeren von Dropropizin
Procédé pour la préparation d'énantiomères de dropropizine

(30) Priority: 24.06.1992 IT MI921554
(43) Date of publication of application: 29.12.1993
(73) Proprietor: BIDACHEM S.p.A., I-24040 Fornovo San Giovanni (Bergamo) (IT)
(72) Inventor: Paradisi, Gianbattista, I-24040 Fornovo san Giovanni (Bergamo) (IT); Bombarda, Carlo, I-24040 Fornovo san Giovanni (Bergamo) (IT); Polacci, Carlo, I-24040 Fornovo san Giovanni (Bergamo) (IT)
(74) Representative: Minoja, Fabrizio, Dr.

(56) References cited:
- EP-A- 0 147 847
- EP-A- 0 349 066
- EP-A- 0 409 044
- FR-A- 2 634 765

## Description

The present invention relates to a new process for the preparation of the enantiomers of the dropropizine, which is the usual name for 3-(4-phenyl-1-piperazinyl)-1,2-propanediol. Other conventional names are laevo-dropropizine for the (S)-3-(4-phenyl-1-piperazinyl)-1,2-propanediol and dextro-dropropizine for the (R)-3-(4-phenyl-l-piperazinyl)-1,2-propanediol, having the following formulas respectively:

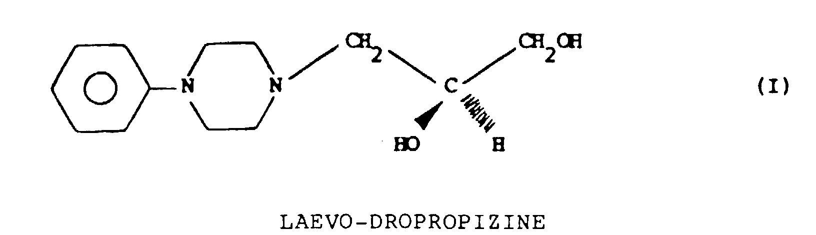

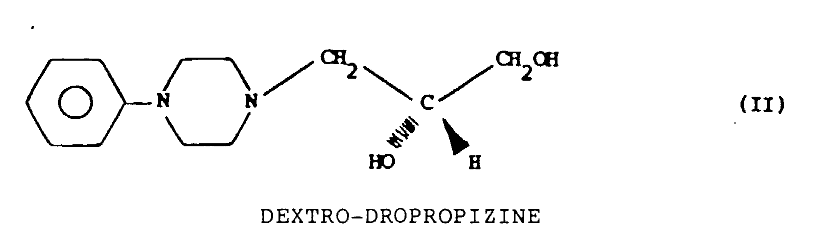

The compounds of formula (I) and (II), as well as their racemic mixture, are known and useful as anti-tussive agents.

According to EP-A-147.847 the compounds of formula (I) and (II) are prepared from racemic dropropizine via optical resolution or are synthetized from (+)-1,2-iso-propylidene-sn-glycerol, which is transformed into the corresponding tosylate and then deketalized. The obtained (-)-glycerol-1-tosylate is condensed with 1-phenylpiperazine to give laevo-form (I).

Similarly dextro-form (II) may be prepared from (+)-glycerol-1-tosylate.

The low yields and the laboriousness due to the process complexity are not satisfactory for industrial applications. Other patents (It. Pat. 1226570, EPA-0349066) describe the preparation of the enantiomers of the dropropizine starting from (R) or (S) glycidols or their derivatives. This way is less complicated in relation to the numbers of the necessary steps, but glycidols and their derivatives are usually known to be unstable compounds and their use on industrial scale often needs some precautions and care for both danger and health.

It has now been found that the use of optically pure 3-halo-1,2-propanediols allows to obtain highly optically pure laevo or dextro dropropizine with high yields. The method of the invention is easily applicable also on industrial scale, because of the lack of problems of stability and handling.

According to this invention the enantiomers of the dropropizine of formula (I) and (II) are prepared by reacting an appropriate (R) or (S) 3-halo-1,2-propanediol with 1-phenyl-piperazine in conformity with the following reactions: 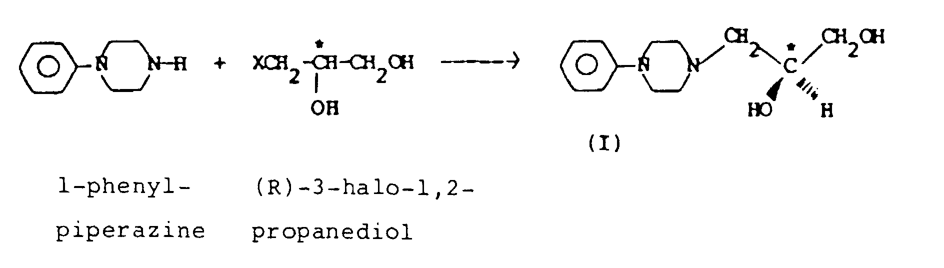 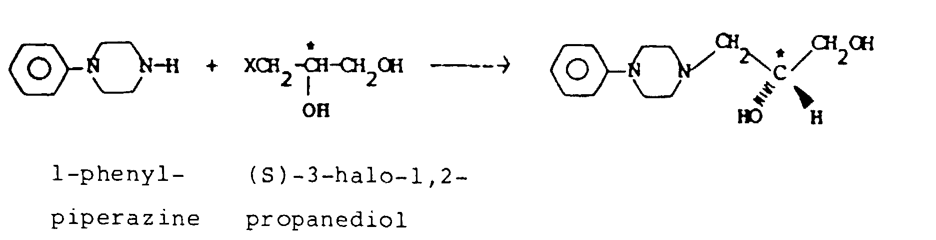 Where X = Cl, Br, I.

The reactions are usually performed in the presence of solvents (or their mixture) and of an organic or inorganic base. The solvents commonly used are methanol, ethanol, isopropanol, toluene or, preferably, water; the base may be an amine, for instance triethylamine or an alkali, for instance sodium or potassium hydroxide. The range of temperature can vary from the room-temperature to the boiling point of the employed solvents. It is particularly preferred the boiling temperature when an organic solvent is used and a range between 40 and 80°C when water is employed, as a solvent. 3-Chloro-1,2-propanediol enantiomers, used as starting material in the above described process, are commercially available.

The optical purity of the enantiomers of dropropizine depends usually on the optical purity of 3-halo-propanediol so that it is also possible to avoid any crystallization in order to get directly very high values (>99%), contrary to the methods, which use glycidols, or their derivatives, and do not generally allow to obtain (R) or (S) dropropizine having optical purity superior to 90% and need one or more crystallizations.

The method of the invention therefore allows to achieve better yields in comparison with the methods described in the previous patents. It is also possible to obtain highly optically pure enantiomers by purifying one or more times in water or solvent the products obtained from low optical grade 3-halo-1,2-propanediols. This makes of course decrease the yields of the process.

However it is preferred to use 3-halo-1,2-propanediols with optical purity higher than 70%. It is particularly preferred the use of (R) or (S) 3-chloro-1,2-propanediol. The invention is illustrated by the following examples.

### EXAMPLE 1

### Preparation of laevo-dropropizine

100 g (0.616 mol) of 1-phenyl-piperazine are mixed
with 74.8 g (0.676 mol) of (R)-3-chloro-1,2-propanediol (optical purity more then 99.5%) and 139 ml of water in a 0.5 1 flask under stirring. The mass is warmed at 60°C and 86.7 g (0.65 mol) of sodium hydroxide are added. After 2 hours at 60°C the mixture is cooled at 0°C overnight and filtered. The collected white crystal is washed with cool water and dried under vacuum, yielding 135.4 g (93%) of laevo-dropropizine.

The optical purity by HPLC is 100%.
M.P. = 102.8°C.

### EXAMPLE 2

### Preparation of dextro-dropropizine

10 g (0.0616 mol) of 1-phenyl-piperazine are mixed with 24 ml of water, 4.72 g of isopropanol, 8.66 g (0.065 mol) of sodium hydroxide and 7.48 g (0.0676 mol) of (S)-3-chloro-1,2-propanediol in a 100 ml flask. The reaction is carried out for 3 hours at 55°C. The alcohol is distilled and the remaining solution is cooled at 20°C overnight. The crystal is filtered, washed with water and dried under vacuum. 13.1 g of dextro-dropropizine are obtained (90%).

The optical purity by HPLC is more than 99.5%.
M.P. = 104.1°C.

## Claims

1. A process for the preparation of laevo-dropropizine and dextro-dropropizine of formula (I) and (II) 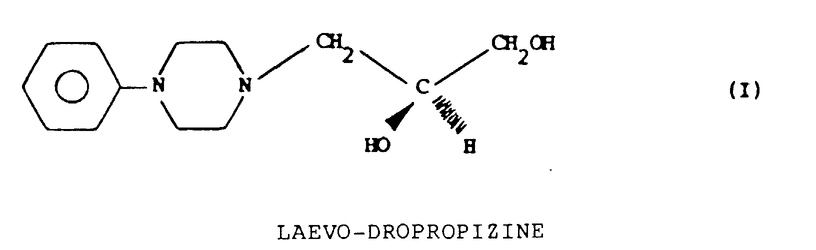 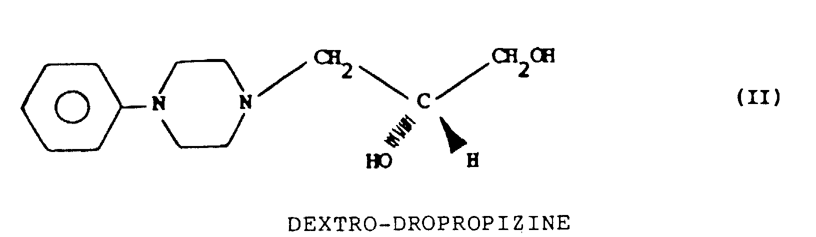 characterised in that 1-phenyl-piperazine is reacted respectively with (S) or (R) 3-halo-1,2-propanediols.

2. A process according to claim 1, characterised in that 1-phenyl-piperazine is reacted with (S) or (R) 3-chloro-1,2-propanediol.

3. A process according to claim 1 or 2, characterised in that the reaction is performed in the presence of water or an inert organic solvent or their mixture.

4. A process according to claim 3, characterised in that the inert organic solvent is selected from methanol, ethanol, isopropanol and toluene.

5. A process according to any one of the previous claims, characterised in that the reaction is carried out in the presence of an organic or inorganic base.

6. A process according to claim 5, in which the base is selected from triethylamine, sodium hydroxide or potassium hydroxide.

7. A process according to any one of the previous claims, characterised in that the reaction is carried out in an organic solvent at the boiling point or in water in the range temperature between 40-80°C.

8. A process according to any one of the previous claims, characterised in that 3-halo-1,2-propanediol has optical purity higher than 70%.

## Patentansprüche

1. Verfahren zur Herstellung von Laevo-Dropropizin und Dextro-Dropropizin der Formeln (I) und (II): 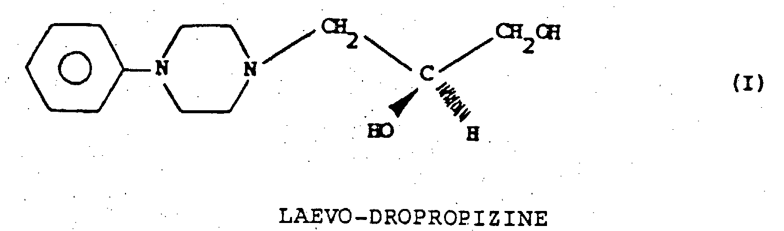 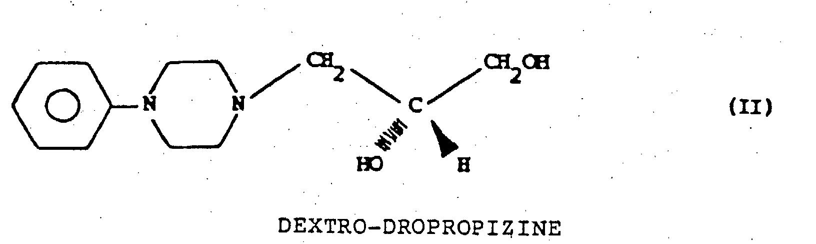 dadurch **gekennzeichnet,** daß 1-Phenylpiperazin jeweils mit (S)- oder (R)-3-Halogen-1,2-propandiolen umgesetzt wird.

2. Verfahren nach Anspruch 1, dadurch **gekennzeichnet,** daß 1-Phenylpiperazin mit (S)- oder (R)-3-Chlor-1,2-propandiol umgesetzt wird.

3. Verfahren nach Anspruch 1 oder 2, dadurch **gekennzeichnet,** daß die Reaktion in Anwesenheit von Wasser oder einem inerten organischen Lösungsmittel oder einem Gemisch davon durchgeführt wird.

4. Verfahren nach Anspruch 3, dadurch **gekennzeichnet,** daß das inerte organische Lösungsmittel ausgewählt wird aus Methanol, Ethanol, Isopropanol und Toluol.

5. Verfahren nach einem der vorhergehenden Ansprüche, dadurch **gekennzeichnet,** daß die Reaktion in Anwesenheit einer organischen oder anorganischen Base durchgeführt wird.

6. Verfahren nach Anspruch 5, dadurch **gekennzeichnet,** daß die Base ausgewählt wird aus Triethylamin, Natriumhydroxid oder Kaliumhydroxid.

7. Verfahren nach einem der vorhergehenden Ansprüche, dadurch **gekennzeichnet,** daß die Reaktion in einem organischen Lösungsmittel beim Siedepunkt oder in Wasser in einem Temperaturbereich zwischen 40 bis 80°C durchgeführt wird.

8. Verfahren nach einem der vorhergehenden Ansprüche, dadurch **gekennzeichnet,** daß 3-Halogen-1,2-propandiol eine optische Reinheit über 70% besitzt.

## Revendications

1. Un procédé pour la préparation de lévodropropizine et de dextro-dropropizine de formules (I) et (II) 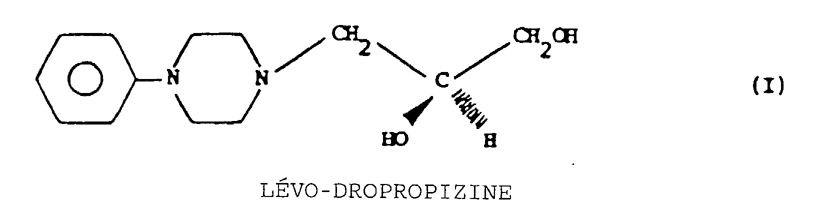 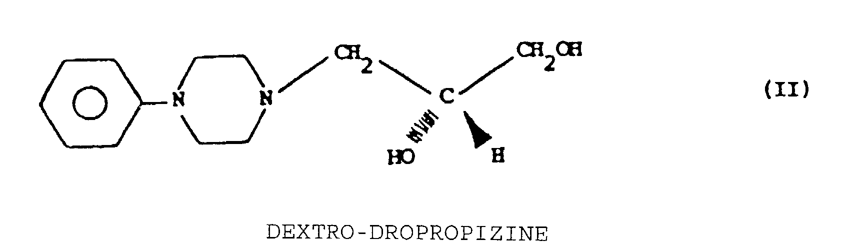 caractérisé en ce qu'on fait réagir la 1-phénylpipérazine avec, respectivement, des (S) - ou (R)-3-halogéno-1,2-propanediols.

2. Un procédé selon la revendication 1, caractérisé en ce qu'on fait réagir la 1-phénylpipérazine avec le (S)-ou (R)-3-chloro-1,2-propanediol.

3. Un procédé selon la revendication 1 ou 2, caractérisé en ce que la réaction est effectuée en présence d'eau ou d'un solvant organique inerte ou d'un mélange d'entre eux.

4. Un procédé selon la revendication 3, caractérisé en ce que le solvant organique inerte est choisi parmi le méthanol, l'éthanol, l'isopropanol et le toluène.

5. Un procédé selon l'une quelconque des revendications précédentes, caractérisé en ce que la réaction est effectuée en présence d'une base organique ou minérale.

6. Un procédé selon la revendication 5, dans lequel la base est choisie parmi la triéthylamine, l'hydroxyde de sodium et l'hydroxyde de potassium.

7. Un procédé selon l'une quelconque des revendications précédentes, caractérisé en ce que la réaction est effectuée dans un solvant organique au point d'ébullition ou dans l'eau dans l'intervalle de température de 40 à 80°C.

8. Un procédé selon l'une quelconque des revendications précédentes, caractérisé en ce que le 3-halogéno-1,2-propanediol a une pureté optique supérieure à 70 %.
